# EUROPEAN PATENT SPECIFICATION

(11) **EP 4 298 430 B1**
(45) Date of publication and mention of the grant of the patent: **17.06.2026**
(21) Application number: 22712128.2
(22) Date of filing: 28.02.2022
(51) Int. Cl.: G01N 21/85, G01N 21/359, G01N 21/47, G01N 33/24

(54) **IN-SITU NEAR INFRARED SENSOR UNIT AND METHOD OF MAKING THE SAME**
IN-SITU-NAHINFRAROT-SENSOREINHEIT UND VERFAHREN ZU DEREN HERSTELLUNG
UNITÉ DE DÉTECTION INFRAROUGE PROCHE IN SITU ET SON PROCÉDÉ DE FABRICATION

(30) Priority: 28.02.2021 US 202117187833
(43) Date of publication of application: 03.01.2024
(73) Proprietor: Landscan LLC, Davis, CA 95616 (US)
(72) Inventor: VAN WYCK, Neal, Edward, San Luis Obispo, CA 93407 (US); ANDERSON, Gregory, San Luis Obispo, CA 93407 (US); FARRINGTON, Stephen, San Luis Obispo, CA 93407 (US); ROONEY, Daniel, James, San Luis Obispo, CA 93407 (US); WALLACE, Woody, Guthrie, San Luis Obispo, CA 93407 (US)
(74) Representative: Ramrath, Lukas
(86) International application number: PCT/US2022/018197
(87) International publication number: WO 2022/183124

(56) References cited:
- DE-A1- 102004 020 350
- US-A- 6 115 061
- US-A1- 2017 370 064

## Description

### RELATED APPLICATION DATA

This application claims the benefit of priority of U.S. Nonprovisional Patent Application Serial No. 17/187,833, filed February 28, 2021, and titled In-Situ Near Infrared Sensor Unit and Method of Making the Same.

### FIELD OF THE DISCLOSURE

The present disclosure relates to the field of near infrared spectrometry. More specifically, it relates to an in-situ NIR sensor unit, especially for sensing components of soil and other media in which it may be positioned.

### BACKGROUND

NIR spectrometry (NIRS) is a reliable testing method utilized by laboratories to determine the constituents of a sample. Test samples are collected in the field, placed in appropriate sample containers and cataloged for future analysis. Once a laboratory receives the samples, each sample container is opened, and a portion of the sample is placed under an NIR sensor machine. The NIR sensor machine uses a light source to illuminate the sample with a broad spectrum of near infrared light such as a wavelength range of about 780 nm to about 2500 nm. In many instances, visible light is also included in the analysis, such as a wavelength range from about 380 nm to about 780 nm, although definitions of the ranges of visible and near-infrared light vary, with some overlap occurring depending on the source of the definition. The sample absorbs, transmits, or reflects light according to, among other things, different bonds which may be present among the constituents of the sample. A spectrometer within the machine sensitive to NIR light, measures the intensity of the light reflected by the sample in each of several wavelength bands. By assessing variations among the bands of reflected NIR light, the laboratory may determine quantities of various compounds present in the sample and may also determine mineral composition of soil grains.

Areas in which NIRS is particularly useful for sample analysis include soil and/or earth related fields including geology, agriculture, and environmental contamination, etc. However, the requirements to collect samples in the field, transport the samples to a laboratory, and waiting for the laboratory to perform the analysis, introduce inefficiencies related to costs, tracking and time lag in receiving the results.

A VIS-NIR equipped soil penetrometer is disclosed in U.S. Patent No. 10,337,159 B2 ("10,337,159"). This penetrometer provides a housing with a conical tip for insertion into soil. Inside the housing, the VIS-NIR sensing components include a light source separate from a fiber optic sensor and a mirror disposed therebetween. The mirror reflects the light emitted from the light source (e.g., lamp or LED) through a window in the housing to the soil external to the penetrometer. Then, light is reflected by the soil through the transparent window into the housing and transmitted by the fiber optic sensor.

Locating a light source within the housing and/or near the sensors such as in 10,337,159 has disadvantages. The light source is separate from the fiber optic sensor and positioned closer to the tip of the penetrometer than the light source. In this position, the light source utilizes space that may be used to add additional sensors which would allow for more accurate NIR sensing. Also, the accuracy of the NIR and any other sensors may be reduced by heat generated by the light source in the area of sample sensing. Moisture content, for example, is particularly useful in interpreting the NIR sensor data. Any moisture sensor incorporated into the penetrometer of 10,337,159 would be exposed to heat from the nearby light source. This may result in reduced accuracy of both readings. For example, a moisture content measurement often includes a measurement of the dielectric permittivity of the soil matrix, which is known to vary with temperature. Introducing an actively heated probe into the soil may induce unknowable and artificial thermal gradients in the soil which will reduce the accuracy of the dielectric-based moisture determination.

Therefore, there is a need for an in situ NIR sensing unit without an internal light source and an improved ability to sense constituents and other features of a media in which the NIR sensor is placed.

DE 10 2004 020350 A1 discloses the use of emission and collection fibers in conjunction with a transparent window in the sidewall of the housing and a mirror.

### SUMMARY OF THE DISCLOSURE

The present invention is directed to a sensing unit as defined in independent claim 1. Preferred embodiments are defined in the dependent claims. One aspect of the disclosure is an in situ NIR sensor or probe which may be inserted into a media and perform in situ NIR sensing. The probe includes various features within a housing such as a sensing element, comprising at least one light collecting fiber and at least one light emitting fiber, mounted opposite a mirror that is mounted at an angle to a transparent window which may be sapphire crystal.

The angle at which the mirror is mounted is beneficial for many reasons. First, it enables the use of near infrared light emitted by an external light source positioned "up hole" above ground and outside of the probe. The light supplied by the external source is conveyed from the source into the probe housing by fibers in a fiber optic bundle. Media may be illuminated via reflection off the mirror and transmission through a transparent window such as a sapphire crystal. Second, the transmission of light to the media occurs while precluding supplied light reflected off the first and second surfaces of the sapphire window from reaching a light collecting fiber in the fiber bundle tip. Light reflected off the media may reach the tip of the light collecting fiber via transmission through the window and reflection off the mirror.

A second aspect of the disclosure is a near infrared sensing unit comprising a tubular housing including a sidewall extending between a first end and a second end, and a transparent window formed in the sidewall of the housing. The sensing unit also comprises a sensing element mounted inside the housing. The sensing element configured to emit near infrared light and configured to collect near infrared light. The sensing unit also comprises a mirror mounted inside the housing and opposite the sensing element. The mirror may be mounted at an angle with respect to the transparent window. The angle allows the mirror to reflect the near infrared light, emitted by the sensing element, through the transparent window. The sensing unit also comprises a conical tip connected to the first end of the housing, and the conical tip is configured to penetrate a media.

The sensing unit comprises a carriage disposed inside the tubular housing with the carriage abutting the sidewall. A mirror mount formed in the carriage, the mirror mount configured to hold the mirror at the angle with respect to the transparent window and to hold the mirror at a specific distance from the transparent window. Also, a sensor mount formed in the carriage, and the sensor mount configured to hold the sensing element opposite the mirror. Additionally, the carriage may be positioned inside the housing such that the mirror and sensing element are adjacent the transparent window.

The sensing unit may further comprise a light source external to the housing; and an optical fiber bundle connected to the sensing element and light source.

The angle of the mirror with respect to the transparent window may be in a range of 33.5° to 38.5°.

The mirror may further comprise a mounting surface facing away from the transparent window, and a reflecting surface facing the transparent window. The transparent window may further comprise a first surface extending parallel to a second surface. A first plane may extend parallel and through the reflecting surface. A second plane may extend through the transparent window such that the second plane is parallel to the first and second surfaces. The angle may be defined by an intersection of the first plane and second plane, and the angle may be designed and configured to result in a predefined angle of incidence of reflected light with the transparent window. In one arrangement the angle of the mirror is in a range of about 33.5° to about 38.5°.

The sensing unit may further comprise a sapphire crystal forming the transparent window. The sapphire crystal may be configured to transmit at least one of ultraviolet, visible, and near infrared light to media exterior to the housing. The sapphire crystal may also be configured to transmit light reflected from the media into the housing.

The sensing unit may further comprise a central opening within the tubular housing. The central opening may be defined by the sidewall, and the central opening may extend between the first end and the second end. The sensing element and mirror may be mounted within the central opening adjacent the window.

The sensing element may further comprise at least one light emitting fiber and at least one light collecting fiber.

The sapphire crystal may include a first surface and a second surface. The first surface facing the interior of the housing, and the second surface facing the exterior of the housing. Also, the sensing element may be mounted in the housing such that the sensing element is configured to emit near infrared light in a path substantially parallel to a plane extending parallel to the first or second surfaces of the transparent window.

The sensing unit may further comprise an optical fiber bundle including a first bundle end comprising the sensing element and a second bundle end. Also, a spectrometer may be external to the housing, and the spectrometer may be connected to the second bundle end. The spectrometer may also be configured to perform a spectral analysis of near infrared light detected by the sensing element, for example, determine variations between the near infrared light emitted by the sensing element and near infrared light detected by the sensing element. Additionally, a light source may be external to the housing, and the light source may be connected to the second bundle end. Also, the light source may be configured to transmit light to the sensing element through a fiber optic bundle.

The angle of the mirror with respect to the transparent window may allow the near infrared light to be transmitted through the sapphire crystal and precludes any near infrared light reflected by the sapphire crystal from impinging on the sensing element.

Described herein, but outside the scope of the present invention, is a method of making a near infrared sensor. The method comprises various steps such as providing a housing including a first end, a sidewall extending between the first and second end, and a transparent window in the sidewall. Also, the method comprises mounting a sensing element inside the housing. The sensing element may be configured to emit near infrared light and detect near infrared light. Another step includes mounting a mirror inside the housing and opposite the sensing element such that the mirror is at an angle with respect to the transparent window and the angle allows the mirror to reflect the near infrared light, emitted by the sensing element, through the transparent window. A further step comprises connecting a conical tip to the first end of the housing, and the conical tip is configured to penetrate a media.

The method of making a near infrared sensor may further comprise the step of forming a carriage configured to be inserted into the housing. The carriage may include a mirror mount configured to hold the mirror at the angle with respect to the transparent window. The carriage may also include a sensor mount formed in the carriage. The sensor mount configured to hold the sensing element opposite the mirror. Another step may include placing the carriage into the housing such that the sensing element and mirror mounts are adjacent the transparent window.

The step of forming the carriage may further comprise forming the mirror mount such that the mirror mount is configured to hold the mirror at an angle with respect to the transparent window.

The step of providing a tubular housing may further comprise the step of using a sapphire crystal as the transparent window.

The step of mounting the mirror may further comprise the step of mounting the mirror at an angle with respect to the transparent window such that the angle of the mirror allows the near infrared light to be transmitted through the sapphire crystal and precludes near infrared light reflected by the sapphire crystal from impinging on the sensing element.

The step of mounting the mirror further comprises the step of mounting the mirror such the mirror is at an angle of about 33.5° to 38.5° with respect to the transparent window.

The step of mounting the sensing element further comprises the step of mounting the sensing element such that the sensing element is configured to emit near infrared light in a path that is substantially parallel to a plane extending through the transparent window and towards the first and second ends of the housing.

The method of making a near infrared sensor may further comprise the steps of providing a light source external to the housing; and connecting the light source to the sensing element with a fiber optic cable bundle. Also, the method may include providing a spectrometer external to the housing and connecting the spectrometer to the sensing element with the fiber optic cable.

### BRIEF DESCRIPTION OF THE DRAWINGS

For the purpose of illustrating the disclosure, the drawings show aspects of one or more embodiments of the disclosure. However, it should be understood that the present disclosure is not limited to the precise arrangements and instrumentalities shown in the drawings, wherein:
FIG. 1 is a side view of an embodiment of a NIR sensor probe according to this disclosure;
FIG.2 is a front perspective view of a NIR sensor housing of the embodiment of the NIR sensor probe of FIG. 1;
FIG. 3 is a longitudinal cross-sectional view of the NIR sensor housing of FIG. 2;
FIG. 4 is a front view of an embodiment of the carriage inside the NIR sensor housing of FIG.2;
FIG. 5 is an exploded view of zone 91, as denoted by dashed line Z1, of the carriage of FIG. 3;
FIG. 6 is a top view of the embodiment of the NIR sensor probe of FIG. 1;
FIG. 7 shows the NIR sensing system of the NIR sensing probe of FIG. 1;
FIG. 8 is an end view of an embodiment of the sensor tip of the NIR sensor in FIG. 7;
FIG. 9 illustrates an example method of making an NIR sensor probe; and
FIG. 10 illustrates an example method of using an NIR sensor probe.

### DETAILED DESCRIPTION

Certain terminology is used in the following description for convenience only and is not limiting. The words "inner", "inwardly" and "outer", "outwardly" refer to directions toward and away from, respectively, a designated centerline or a geometric center of an element being described, the particular meaning being readily apparent from the context of the description. Also, as used herein, the words "connected" or "coupled" are each intended to include integrally formed members, direct connections between two distinct members without any other members interposed therebetween and indirect connections between members in which one or more other members are interposed therebetween. The terminology includes the words specifically mentioned above, derivatives thereof, and words of similar import.

Throughout the present disclosure, unless otherwise specified, the term "about" when used with a corresponding numeric value may refer to ±20% of the numeric value, or may refer to ±10% of the numeric value, or may refer to ±5% of the numeric value, or may refer to ±2% of the numeric value.

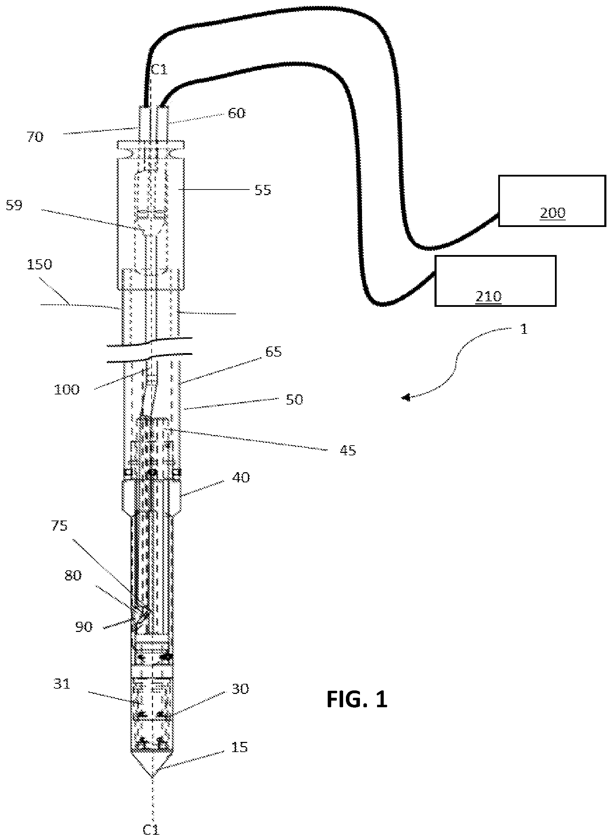

Like numbers are used to indicate like elements throughout. Elements, components, and/or features that are discussed herein with reference to one or more of FIGS. 1- 10 may be included in and/or utilized with any of FIGS. 1-10, wherein in general, elements that are likely to be included in a particular embodiment are illustrated in solid lines.

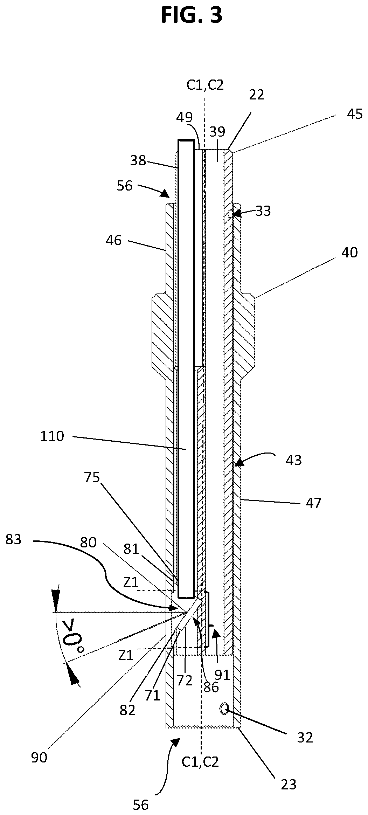

Figs. 1-10 provide examples, according to this disclosure, of a sensing unit that includes an in situ NIR probe 1, method of making the in situ NIR probe 1 and the method of using the in situ NIR probe 1. The NIR probe 1 includes an NIR sensing element 110 (see FIG. 3) and may perform in situ sensor readings allowing the NIR sensor readings to be taken at a field location. Additionally, the probe 1 may include other sensors such as soil force sensor 30 incorporated into sleeve 31 and more. More specifically, the NIR probe 1 is configured to be inserted into a media 150, such as soil and other geologic elements, and perform NIR spectrometry readings. The readings may be performed at various depths below a surface of the media 150 adjacent and/or surrounding the sensing element 110 as well as perform NIR sensor and soil force readings as the probe 1 is inserted into the media 150. This allows soil force data to be obtained as the probe 1 is inserted and the NIR sensing to be performed on the same media 150.

It is noted that the NIR probe 1 may also perform sensing when the probe is not inserted in soil or other media by positioning a sample sufficiently near the sensing element 110.

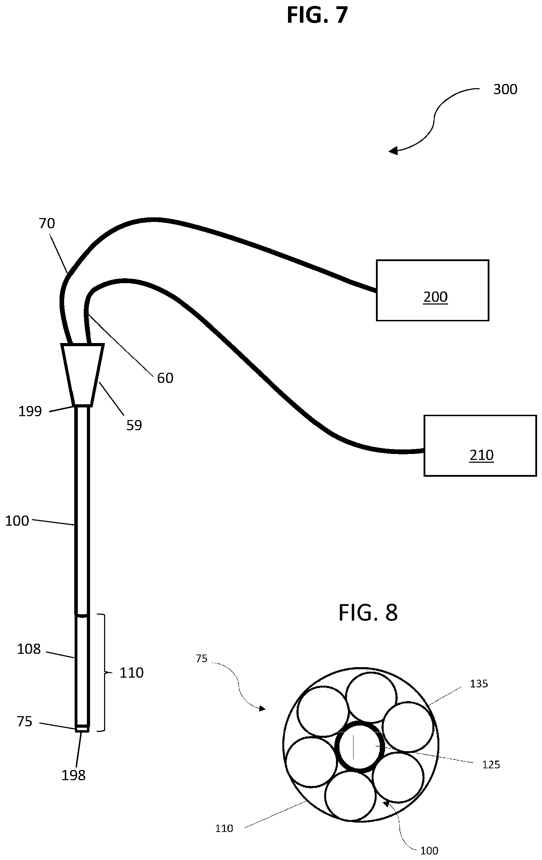

As shown in FIGS. 1-8, the NIR probe 1 includes a housing 65 with a window 90, a mirror 80, a fiber bundle 100 with a NIR sensing element 110 including sensing element tip 75, a spectrometer 200, a light source 210 and a bracket or carriage 45. The NIR sensing occurs within the housing 65 via NIR sensing element 110 and corresponding tip 75 which are part of an NIR system 300, as shown in FIG. 7, including a spectrometer 200 and light source 210 connected to the sensing element 110 by a fiber bundle 100 that includes separate fiber bundles 70 and 60 connected to the spectrometer 200 and light source 210, respectively.

The housing 65 may include multiple in-line sections such as probe tip 15, sleeve 31, sensor housing 40, push rod 50, and end cap 55. Sleeve 31, sensor housing 40, push rod 50, and end cap 55 are tubular and formed about a centerline C1. In the illustrated embodiment, the sensor housing 40 has a rectangular or square tubular cross section and the other sections 31, 50, 55 include annular cross-sections. The tip 15 may be formed for ease of insertion into a media such as soil, wells, earth, etc. and may have various shapes and/or cross sections including conical, tapered, pointed, or triangular, and the tip 15 may also be formed about centerline C1.

The sensor housing 40 is connected to the push rod 50 and the sleeve 31. One end of housing 65 is formed by the tip 15 which is connected to the sleeve 31. The other end of the housing is formed by the end cap 55 which is connected to the push rod 50. Sections 15, 31, 40, 50, 55 may be integrally formed or separate sections that are connected through suitable methods such as adhesives, opposable threaded fasteners, rivets, screws, epoxies, etc. Additionally, sections 15, 31, 40, 50, 55 may be formed of the same or similar materials that are resistant to deformation upon insertion into the media. For example, suitable materials may be three dimensionally stable materials including hard plastics, thermoplastics, metallic compounds or alloys (i.e., aluminum, stainless steel), ceramics, hard plastics, etc. Each section may be formed by a process corresponding to the chosen material such as metal milling, CNC machine, casting, ceramic molding, injection molding and additive manufacturing, etc.

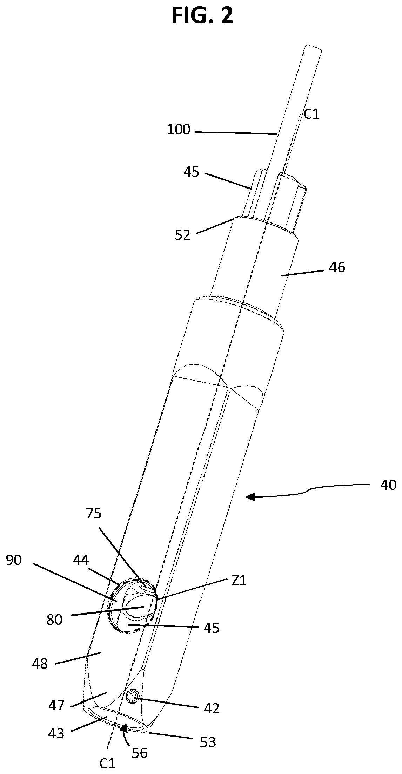

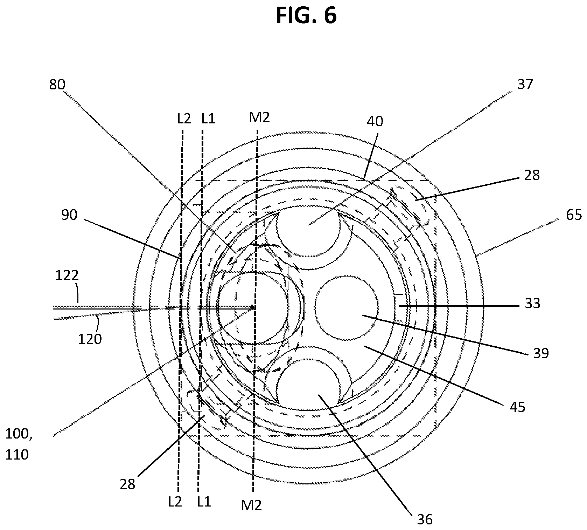

The tip 15 abuts and is connected to the sleeve 31 by threaded fasteners or other methods as described above. Sleeve 31 may include a soil force sensor 30 and other types of sensors. The sleeve 31 may be connected to the sensor housing 40 via threaded or other fasteners 28, as shown in FIG. 6, inserted into through holes 42, as shown in FIG. 2) in the sensor housing 40.

The sensor housing 40 is shown in FIGS. 2-3 and comprises a tubular wall 48 having an exterior surface 47 and an interior surface 43 extending between a first end 52 and a second end 53. The interior surface 43 surrounds and defines a central opening 56 which also extends from the first end 52 to the second end 53. The first and second ends 52, 53 may be relatively smaller in diameter and/or width than a section of wall 48 therebetween. At the first end 52, sleeve section 46 is formed for insertion into the push rod 50. The second end 53 tapers for connection with the sleeve 31. Through hole 42 positioned on the second end 53 allows connection to the sleeve 31 with a fastener 28 such as a pin, ribbed fastener or threaded fastener. Additionally, the sleeve 46 and second end 53 may have features (not shown) such as threading and/or ridges for connection with the push rod 50 and sleeve 31.

An aperture 44 extends through the housing wall 48 and the exterior and interior surfaces 47, 43, respectively. A transparent material, such as a sapphire crystal or other material with suitable light transmissivity in the NIR spectral range, strength, and hardness, is disposed in and fills the aperture 44 and serves as a transparent window 90 in the wall 48 between the sensing element tip 75 and external media 150. The window 90 may be secured in the aperture 44 by using a marine adhesive or other suitable elements including epoxies, adhesives and/or a bracket, bezel, etc.

The carriage 45 may be disposed within the sensor housing 40, as shown in FIG. 3, and the carriage 45 is configured to hold the NIR sensing element 110, mirror 80 as well as other sensors and cables. To permit sensing through window 90, the carriage 45 is secured in position such that the sensing element tip 75 and mirror 80 are adjacent and directly opposite the window 90. More specifically, the mirror 80 and sensing element tip 75 may be within zone 91 which is denoted by dashed line Z1. The carriage may be secured to the housing 40 by inserting a set screw into securing hole 33, but other types of fasteners such as adhesives, epoxies, and/or hook and loop may be utilized, or its position may be fixed by fastening it to other components or sensors which are disposed within and secured to the sensor housing 40.

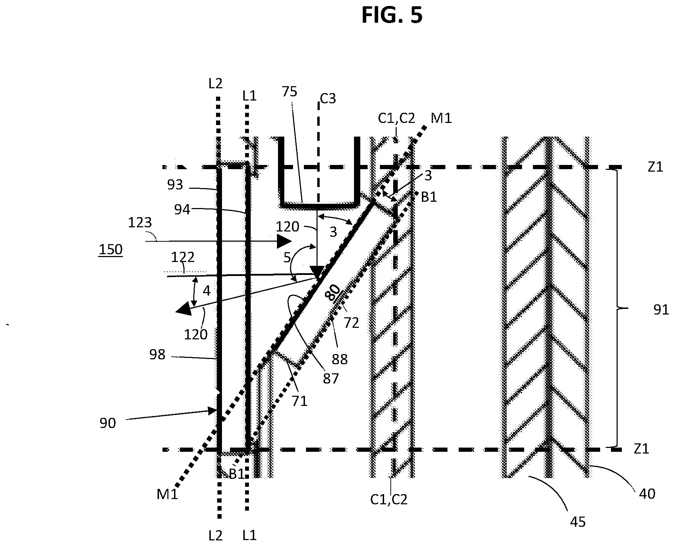

Line Z1 surrounds the perimeter of window 90. Zone 91 extends within the boundaries of line Z1 surrounding the perimeter of the window surface 93 and into the housing 40 to the centerline C2 (see, e.g., FIG. 5). This zone 91 defines the three-dimensional zone in which the sensing element tip 75 and mirror 80 may be placed so the light 120 emitted from the sensing element tip may be reflected by the mirror 80 through the window 90 to media 150. In the illustrated example, the mirror and sensing element tip are laterally offset from a central longitudinal axis of the tubular housing and positioned directly laterally adjacent the transparent window.

In the illustrated example window 90 is circular, but it is envisioned the window may be other shapes including square or rectangular. If the window 90 has different shapes then line Z1 would surround the perimeter of the window 90 and create a correspondingly shaped zone. In the illustrated example, zone 91 is a space envelope defined by the window 90 that extends in a lateral direction across a width of the tubular housing and extends in an axial direction from a top edge of the window to a bottom edge of the window and the sensing element tip 75 and mirror 80 are located within the space envelope.

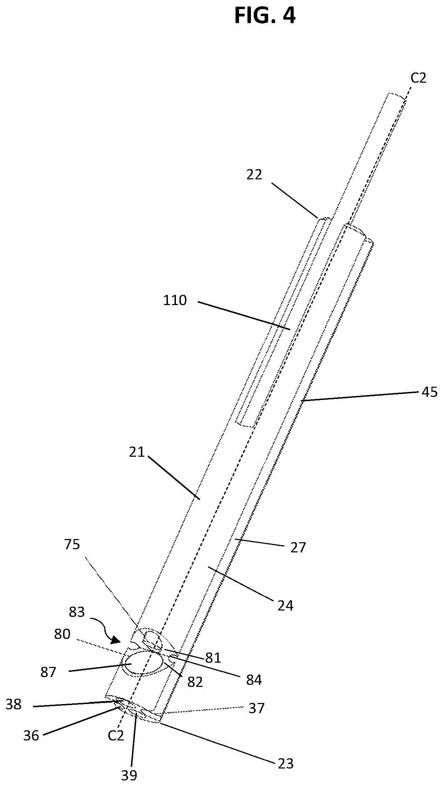

As shown in FIGS. 3 and 4, the carriage 45 has a centerline or central axis C2 which is coaxial and/or parallel to centerline C1 of the housing 40 and a body 21 with an exterior surface 24 extending between a first end 22 and a second end 23. Also, the carriage may include a mirror recess 83, securing hole 33, sensor wire channels 36, 37 and sensor mounts 38, 39. The first end 23 is positioned within the first end 52 of housing 40 and the second end 22 may extend beyond the first end 52 of housing 40. The exterior surface 24 may abut and be contiguous with the interior housing surface 43.

The carriage 45 may support several sensors. In this embodiment, the carriage includes sensor mounts 38 and 39 configured to hold sensors and/or sensing elements as well as wire channels 36 and 37 configured to hold sensor wiring. As shown, mount 39 is a through hole while channels 36 and 37, which also extend the length of the carriage 45, are defined by the external surface 24 with an opening 27 extending along the sides from the first end 23 to the second end 22 of the carriage 45. In this case, mount 38 holds the NIR sensing element 110 and channel 37 would hold any wiring (not shown) associated with a different sensor such as the soil force sensor 30. It is envisioned that channels 36, 37 and mounts 38, 39 may each hold a sensor/sensing element and/or allow wires to pass through the carriage 45. Additionally, it is envisioned that more or fewer mounts, including varying dimensions, may be formed in carriage 45.

Mount 38 is a modified elongate longitudinal opening that extends along at least a portion of a length of the carriage 45. In the illustrated example a central axis of at least a portion of mount 38 is parallel to central axis C2. In other examples, at least a portion of mount M2 may be angled with respect to central axis C2 and not parallel to axis C2 for positioning at least a portion of sensing element 110 at an acute angle with respect to axis C2. In the illustrated example mount 38 is a through hole as it appears open on the carriage first end 22 and second end 23. However, the mirror recess 83, as shown in FIGS. 3-5, is formed in the mount 38, carriage body 21, and exterior surface 24. Mirror recess 83 is a notched opening formed in exterior surface 24 of the carriage 45 that extends partially through a width of the carriage from exterior surface to, e.g., centerline C2 of the carriage. The recess 83 may be defined by a first surface 81 opposite and abutting a second surface 82 with the first and second surfaces 81, 82 extending outwardly and radially with respect to the centerline C2. At least first surface 81 intersects the sensor mount 38. An angle of surfaces 81 with respect to, e.g., centerline C2, may vary. In the illustrated example, surface 81 is at an acute angle with respect to centerline C2 which allows for sensing element tip 75 to protrude into recess 83 and increases an interior volume partially defined by recess 83 to increase an amount of reflected light from a soil sample that reaches the sensing element tip. In other examples, surface 81 may be an angle that is about orthogonal to centerline C2. Additionally, the first surface 81 may extend axially towards the first end 22 and the second surface 82 may extend axially towards the second end 23. As shown, surfaces 81 and 82 may abut and may be contiguous at joint 84.

A mount recess 86 (FIG. 3) may be formed in the second surface 82. The mount recess 86 is configured to hold the mirror 80 with a bottom surface 72 and a sidewall 71. In an example, bottom surface 72 is substantially parallel to second surface 82 and sidewall 71 is at a right angle to bottom surface 72 and has a complementary shape to an outer perimeter of mirror 80 to locate the mirror in the mount recess. Like surface 82, bottom surface 72 extends radially and axially towards the second end 23. Additionally, the bottom surface 72 may extend entirely across the mount recess 86 or a portion of the mount recess 86. To ensure the mirror 80 is properly oriented towards the window 90, the bottom surface 72 is at an angle 3, as shown in FIG. 5 to a centerline C3 of sensing element 110. Angle 3 extends between a plane B1 extending through and parallel to surface 72 and centerline C3. Due to the angle 3 of the bottom surface 72 on which the mirror 80 is mounted, a plane M1 extending through and parallel to mirror 80 is also positioned at the angle 3 to the centerline C3. In the illustrated example, window 90 extends parallel to the centerline C3 so angle 3 also extends between plane M1 and planes L1 and L2 extending across and parallel to window surfaces 94 and 93, respectively.

The mirror 80 has a reflective mirror surface 87 and mounting surface 88. In the illustrated example the mirror is flat, however, in other examples, other mirrors known in the art, such as a parabolic mirror, may be used. The mirror surface 87 faces both the sensing element tip 75 and window 90. Mounting surface 88 faces bottom surface 72 and away from the window 90. The mirror surface 87 and angle 3 are designed and configured for the mirror 80 to reflect light 120 emitted by sensing element tip 75 towards the window 90 for efficient transmission through the window to the media 150. The mirror 80 may be secured to surface 72 with a marine adhesive or other suitable elements including bonding agents (i.e., epoxies, adhesives) and/or fasteners. In the illustrated example, probe 1 does not include additional optical components, such as lenses, in the path between sensing element tip 75 and media 150. Such an arrangement allows for a compact design where sensing element tip 75 can be positioned closely adjacent to mirror 80 and both the sensing element mirror can be positioned closely adjacent to window 90, thereby shortening the optical path between sensing element tip and the media and minimizing the space envelope that the window, mirror and sensing element tip encompass. The compact arrangement, with the optical components laterally offset from central longitudinal axes C1, C2 is also an efficient use of space within the probe that allows for incorporation of other sensing elements in the probe. In other examples, additional optical components, such as lenses and filters, may be incorporated into the optical path of the NIR light 120.

In the illustrated example mirror 80 and reflective mirror surface 87 are suitable for reflection of light in the visible and NIR spectrum. One example of a suitable mirror includes a protective gold mirror (e.g., sputtered or evaporated gold) coated with a thin protective dielectric film which may be a thin layer or multiple layers of a dielectric materials such as amorphous SiO2 or Si3N4 (silicon nitride). This type of mirror 80 has a reflectivity of about > 96% across the NIR spectral range, e.g., a spectral range of from about 780nm to about 2500nm.

Figure 5 depicts an enlarged view of zone 91 which extends between lines Z1 and Z1. For ease of illustration, lines Z1 are shown extending through the carriage 45, but as stated above zone 91 is bounded by line Z1, the window first surface 94 and centerline C2. As shown, the mirror 80, sensing element tip 75 and window 90 are disposed in zone 91. Due to the mirror angle 3, the mirror 80 may reflect light 120 emitted from the sensing element tip 75 towards window 90 at an angle 4 with respect to the window that is designed and configured for efficient transmission through the window to media 150. Media 150 reflects some portion of the light 121 as diffusely reflected light 123. Reflected light 123 is conceptually shown as a single arrow orthogonal to window 90, however, the reflected light is diffuse and incident on the window across a range of angles. A portion of the reflected light 123 passes through the window 90 and is incident on sensing element tip 75 and then transmitted through one or more light collecting fibers 135 (FIG. 8) to a spectrometer, e.g., spectrometer 200, for spectral analysis.

Light 120 may be reflected from the mirror 80, through the window 90, to the media, with the angle of the mirror 80 being in a relatively large range. However, at some angles, a substantial proportion of light 120 incident on window 90 may be reflected by the first surface 94 and/or second surface 93 of the window rather than refracted and transmitted therethrough. These unwanted reflections may enter the sensor housing 40 and reach the sensing element tip 75 negatively impacting the ability of the probe to detect the signal of interest, i.e., light 123 reflected from the media 150 that passes through the window 90 to the sensing element tip 75. When this occurs, the light reflected by window 90 may cause the NIR spectral analysis performed by probe 1 to be inaccurate. As a result, in some examples, probes made in accordance with the present disclosure may have a mirror 80 that is positioned at an angle 3 with respect to central longitudinal axis C3 of sensing element 110 that is designed, configured, and selected to preclude or minimize the unwanted reflection of the light 120 by first and second surfaces 94, 93. By way of example, for some window materials, such as sapphire, if light 120 incident on window 90 is orthogonal to the window surface 94 (if angle 4 is 0°), a substantial proportion of the light will be reflected rather than transmitted. On the other hand for arrangements that result in light 120 having a relatively high value of angle 4, such an arrangement may also result in an insufficient proportion of the light 120 being transmitted through the window. In the illustrated embodiment, angle 3 is designed, configured and selected to minimize unwanted reflections from window 90 while maximizing a proportion of light transmitted through the window to increase a signal to noise ratio of the media NIR signal associated with reflected light 123.

Angle 3 illustrated in FIG. 5, defining an angle between mirror 80 and sensing element 110, corresponds to the arrangement shown in FIG. 5 where sensing element 110 is positioned substantially parallel to window 90 with light 120 emitted by sensing element tip 75 similarly traveling in direction that is substantially parallel to the window. An angle between mirror 80 and window 90 is, therefore, the same as angle 3 in the illustrated example. In other examples, such as where sensing element tip 75 emits light 120 at another angle relative to window 90, angle 3 of mirror 80 relative to axis C3 may be the same while an angle of mirror 80 relative to window 90 may be correspondingly adjusted to an angle that is different from angle 3 to achieve the desired angle 4 of light 121 relative to window 90.

To preclude or minimize the proportion of light reflected by either the first or second surface 94, 93 from being incident on sensing element tip 75, a position of mirror 80 may be designed and configured so that the light 120 is incident on the first surface 94 of the window at an angle 4 in a range of about 14° to about 18°, and in some examples, about 16° from normal 122. With an angle of incidence 4, in the range of, e.g., about 14° to about 18°, the proportion of light reflected by window 90 may be substantially reduced or eliminated.

With an angle of incidence 4 in the range of about 14° to about 18°, the angle 5 between the light 120 emitted from the sensing element tip and the light 121 reflected by the mirror 80 is in an approximate range of about 104° to about 108°, and more specifically about 106°.

In the illustrated example, to achieve the angle of incidence 4 in the range of about 14° to about 18°, the mirror 80 is mounted at angle 3 with a range of about 35° to about 39° between the plane M1 and a central longitudinal axis C3 of sensing element 110. In the illustrated example, at least an end portion of the sensing element is positioned in a substantially parallel relationship to centerline C2 and so the angle between M1 and C3 is the same as the angle between the plane M1 of the mirror surface 87 and planes L1, L2 of window 90. To achieve the angle of incidence of about 16°, angle 3 may be about 37°. Therefore, mounting surface 72 within recess 86 may be formed such that it is at an angle 3 of about 35° to about 38° and in some examples about 37° from the centerline C3 of the sensing element, and in some examples, also about 37° from the centerline C2 of the carriage 45. In other examples where sensing element 110 is not parallel to plane L1 of the first surface 94 of window 90, an angle of the mirror 80 relative to centerline C2 may be in a correspondingly different range so that the light 120 reflected by the mirror has the desired angle of incidence 4 with respect to the window. In an example, at least a light emitting end portion of sensing element 110 may be disposed at an angle within the carriage, for example, an angle between axes C2 and C3 may be in the range of about 1° to about 15°, and in some examples, in the range of about 2° to about 10°, and in some examples, about 5° and in some examples, about 2°. In such examples, an angular position of the mirror within the carriage may be adjusted so that the angle 3 between the mirror and the sensing element may be the same as in the illustrated example and an angle between the mirror surface 87 and the window (e.g. the angle between plane M1 and planes L1, L2) may be different. By way of non-limiting example, in an implementation where the angle between axes C2 and C3 is greater than zero and the sensing element 110 is pointing or tilted towards the window 9, an angle of the reflecting surface of the mirror relative to the first and/or second parallel surfaces of a transparent window may be greater than angle 3. In examples where the sensing element 110 is pointing or tilted away from the window 9, an angle of the reflecting surface of the mirror relative to the first and/or second parallel surfaces of a transparent window may be less than angle 3.

When light 123 is reflected from media 150, the light 123 may be transmitted into the sensor housing 40 through window 90 and reflected off of mirror 80 to the sensing element tip. The angle 3 of mirror 80, which in some examples may be in the range of about 35° to about 39°, may be designed and configured to also direct light 123 towards the sensing element tip 75. The foregoing angles are provided by way of example and may be varied according to a particular application. The foregoing angles may be suitable for a window material that is sapphire or another material with optical properties, such as index similar to sapphire and for NIR light 120 that has a wavelength in the range of about 750 nm to about 2500 nm or about 780 nm to about 2500 nm, or a portion thereof. The foregoing angles may also be suitable for light 120 that has a wavelength range that includes visible light, e.g. from about 380 nm to about 780 nm. Thus, in an example, an angle of the mirror is designed and configured to cause the near infrared light emitted by the sensing element to be reflected from the mirror and incident on a surface of the window at an acute angle with respect to an axis that is normal to the surface of the window, the acute angle designed and configured according to a wavelength of the near infrared light and a material type of the window to minimize a reflection of the near infrared light by the window while maximizing a transmission of the near infrared light through the window.

Figure 6 is a top cross sectional view of probe 1 taken at the top of push rod 50. As shown, the fiber bundle 100 and sensing element110 are within mount 38 of carriage 45. For ease of illustration, channels 36 and 37 are empty, however, in use, soil force sensor 30 and/or other sensors may be disposed in the probe and wiring and other components of the other sensors may be located in one of channels 36 or 37. Additionally, set securing hole 33 is shown for the connection of the carriage 45 to the housing 40, and fasteners 28 for attaching the housing 40 to sleeve 31 via through holes 42 are shown.

Further, the mirror 80 is laterally centered opposite the window 90 and is not rotated with respect to window 90. More specifically, a line M2 (FIG. 6) extending across the mirror surface 87 and contiguous with the mirror surface 87 is substantially parallel with planes L1 and L2.

Figures 7 and 8 depict an example, according to this disclosure, of a sensing system 300 that is designed to be operably coupled to a soil penetrometer such as probe 1. As shown, the fiber bundle 100 may be an assembly including a protective cover surrounding a co-aligned arrangement of optical fibers capable of transmitting light in a spectral range that includes at least one of ultraviolet light (UV) in a range of about 200 nm to about 400 nm, visible (VIS) light in a range of about 380 nm to about 780 nm, and/or NIR light in a range from about 780 nm to about 2500 nm. The fiber bundle 100 extends between a first bundle end 198 and a second bundle end 199. The first bundle end 198 includes sensing element tip 75 and sensing element 110. The fibers 125 and 135, which may be arranged as shown in the sensing element tip 75 of Figure 8, may include a single light collection fiber 125 surrounded by a plurality, e.g., six illumination fibers 135. It is noted that the size, material type and numbers of fibers 125 and 135 may vary. For example, in some examples, fiber bundle 100 may include a plurality of light collection fibers 125 that are formed from different materials that are optimized for transmission of different wavelengths of light and are connected to different sensors in one or more spectrometers 200 for spectrally analyzing the different wavelength ranges. For example, a first light collection fiber configured to transmit light with a wavelength below about 1000 nm and connected to a first sensor, e.g., a CCD sensor, for spectral analysis of the light. And a second light collection fiber configured to transmit light above about 1000 nm and connected to a second sensor, e.g., an InGaAs or PbS based sensor, for spectral analysis of the light. In other examples a single light collection fiber 125 may be connected to two or more sensors that each analyze a subset of the entire wavelength range of light transmitted by the light collection fiber. Illumination fibers may similarly be formed from different materials and/or connected to light sources having different spectral characteristics. The fiber bundle 100 is surrounded by a tubular collar 108 which protects and binds fibers 125 and 135 together and assists in holding the tip in the proper position within the carriage 45. The tubular collar 108 may be formed of a material with suitable hardness and three-dimensional stability.

Examples of a suitable material include aluminum, stainless steel, metallic alloys, hard plastics, ceramics, etc.

As shown in Figures 3 and 6, the sensing element 110 including sensing element tip 75 and tubular collar 108 is inserted into mount 38 of the carriage 45 and extends the length of the carriage 45. This length provides stability to the sensing element tip 75, but the collar 108 may be of varying lengths as long as the collar 108 maintains stability of the sensing element tip 75. Mount 38 holds sensing element 110 in a position which allows the illumination fibers 135 to emit light 120 in a desired direction, for example, substantially parallel to the first and second surfaces 94, 93 of the window 90 and corresponding planes L1, L2 as well as centerlines C1, C2. In other examples, light 120 emitted by sensing element tip may be at an acute angle relative to window 90. Sensing element 110 may abut an interior surface 49 of the mount 38 and be held in place by various methods including a friction fit, epoxy, adhesives, fasteners, etc.

As shown in Figures 1 and 8, the fiber bundle 100 extends out of collar 108, carriage 45, and housing 40 through the push rod 50 into a fiber bundle divider 59 which may be disposed in the end cap 55. The fiber bundle divider 59 separates the illumination fibers 135 from the light collection fiber 125. The light illumination fibers 135 then form fiber bundle 60 which is connected to light source 210. The light sensing fiber 125 then forms fiber bundle 70 which is connected to the spectrometer 200. Both fiber bundles 60 and 70 are essentially fiber assemblies including optical fibers which are surrounded by a protective jacket.

The light source 210 and spectrometer 200 are both external to the probe 1 and connected to the sensing element 110 by fiber bundles 60, 70 and 100. The fiber bundle 100, light source 210 and spectrometer 200 may be designed configured and selected according to meet the goals of sensing. For example, light source 210 may be configured to emit light in a spectral range that includes at least one of ultraviolet light (UV) in a range of about 200 nm to about 400 nm, visible (VIS) light in a range of about 380 nm to about 780 nm, and/or NIR light in a range from about 780 nm to about 2500 nm and spectrometer 200 may similarly be configured to analyze light in one or more of the UV, VIS, and NIR spectral ranges.

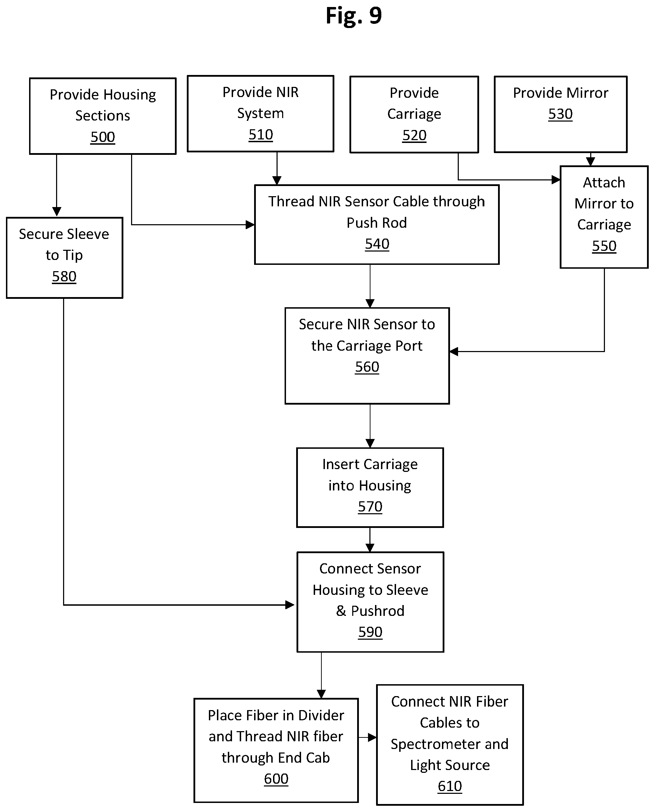

Figure 9 depicts an example of a method of making the NIR probe 1 according to this disclosure. Initially, in steps 500, 510, 520, and 530 different aspects of the NIR probe 1 are separately provided. In step 500, the housing 65 is provided. As described above, the housing 65 includes tip, 15, sleeve 31, sensor housing 40 including sapphire window 90, push rod 50, and end cap 55. Each section 15, 31, 40, 50 and 55 are tubular, formed about a centerline C1, and formed so as to be connected to inline. Also, sensor housing 40 has a central opening 56 that is large enough to allow the carriage to slide through. Additionally, the length of the push rod 50 maybe altered to accommodate the depths at which NIR sensing may be desired. As discussed above, each part may be formed of dimensionally stable materials and as appropriate by processes such additive manufacturing, injection molding, casting, metal forming and milling, etc.

Separately, in step 510, the NIR system 300 is provided. As discussed above, this includes at least the sensing element 110 with sensing element tip 75 with collar 108, fiber bundle divider 59, fiber bundles 60, 70 and 100 and an external light source 210 and spectrometer 200.

In step 520, the carriage 45, as described above, is provided. The carriage 45 is sized such that it may be inserted into central opening 56 in the sensor housing 40. Also, although the carriage 45 may have different mounts 38, 39 and channels 36, 37, for the example of the NIR probe 1 according to this disclosure, the carriage includes at least one mount 38 configured to receive the NIR sensing element 110. Recess 83 is also formed in the carriage 45 and includes a mounting recess 86 formed in mount 38. The mounting recess includes a bottom surface 72 at an angle 3 to the centerline C3 of the sensing element 110. Angle 3 may be in the range of about 33.5° to about 38.5° and in some examples about 37.5°.

The carriage 45 may be formed of dimensionally stable materials including hard plastics, metals, metallic compounds, ceramic, fiberglass, etc. Additionally, the carriage may be formed by suitable processes including additive manufacturing, injection molding, casting, metal forming and milling, etc.

In step 530, a mirror 80 such as a protective gold mirror, as described above, and sized appropriately for mounting on in the mount recess 86 is provided.

Next, assembly of the probe 1 begins in three separate steps. In step 540, fiber bundle 100 including sensing element 110 may be threaded through the push rod 50. In step 550, the mirror 80 is securely attached, as described above, to the bottom surface 72 of mirror recess 83. Once attached, the mirror 80 is at approximately the same angle 3 as the bottom surface 72. Angle 3 is shown in FIG. 5 and discussed above as being approximately in the range of about 33.5° to about 37.5° and in some examples about 37.5°. In step 580, the sleeve 31 is attached to the tip 15 by any of the methods provided above.

In steps 560, the carriage 45 is further prepared for insertion into housing 40. The NIR sensing element 110, which was threaded through the push rod 50 in step 540, is threaded into mount 38 such that the sensing element tip 75 is directly opposite the mirror 80 and the sensing element tip is able to emit light in a trajectory substantially parallel to the centerline C2 of the carriage. It is noted that the accuracy of the sensor readings may increase as the sensing element tip 75 is positioned closer to the mirror 80. As such the optimal position may include placing the sensing element tip 75 as close as possible to the mirror 80 without allowing the sensing element tip 75 to touch the mirror 80. The sensing element 110 is secured in the chosen position by methods described above.

In step 570, the carriage 45 with the NIR sensing element 110, and mirror 80 may be inserted or slid into the sensor housing 40 and secured in the proper position. As discussed above, the carriage 45 should be oriented so the mirror 80 and sensing element tip 75 are within zone 91 and directly opposite the window 90. Further, the mirror 80 should face the window 90 such that the line M2 which is contiguous with mirror surface 87 is substantially parallel to planes L1 and/or L2 of the window surfaces 94, 93, respectively. In other words, the mirror 80 should not be rotated with respect to the window. As discussed above, a set screw may be inserted into securing hole 33 and may be used to secure the carriage 45. The set screw offers the benefit of being able to remove the carriage 45 from to the sensor housing 40, but other suitable methods may also be used.

In step 590, sensor housing 40 is connected to additional elements of the housing. The housing second end 53 is connected to sleeve 31, which was connected to the tip 15 in step 580. This connection may be made using different connection element described above including fasteners 28 being inserted into through holes 42 in sensor housing 40. Additionally, the housing first end 52 is connected to the push rod 50, in which the fiber bundle 100 was previously inserted.

In step 600, the end cap 55 is applied to the push rod 50. This is accomplished by threading fiber bundles 60 and 70 through the opening in the end cap 55. Next, the fiber bundle divider 59 is attached to fiber bundles 60, 70 and 100 such that the fiber bundle 60 includes at least one light illumination fiber 135, fiber bundle 70 includes at least one light sensing fiber 125 and fiber bundle 100 includes at least one light illumination fiber 135 and at least one light sensing fiber 125. The end cap 55 is then slid over divider 59 and the top of the push rod. Additionally, the end cap 55 may be secured to the push rod 50 by opposing threaded fasteners or other methods discussed above.

In step 610, the probe 1 is connected to the spectrometer 200 and light source 210. This connection is made by connecting fiber bundle 70 to spectrometer 200 and fiber bundle 60 to light source 210.

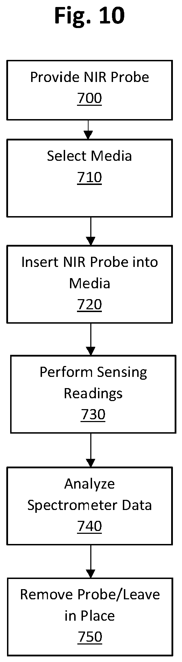

Figure 10 provides an example of the steps involved in a method of using the NIR probe 1 according to this disclosure. Initially, in step 700, an NIR probe 1 as described in this disclosure is provided. Next, in step 710, the media 150 to be sampled is selected. The probe 1 may be used to sense characteristics of various types of media 150 including soil for agricultural and environmental studies, stored grain, compost, bore holes, etc.

In step 720, the probe 1 is inserted into the media 150. Depending on the condition of the media 150, this may involve insertion by hand, with the use of tools and/or machinery such as a hydraulic press.

In step 730, NIR sensing readings may be performed on the media 150 as the probe 1 is inserted and the probe reaches a depth where the window 90 is submerged or in contact with the media 150. In other words, the sensing may be performed at the desired depth of insertion and as the probe 1 reaches the depth.

To perform NIR sensing, the external light source 210 and spectrometer 200, which are both on the surface of the media 150, are activated. Light 120 is transmitted through fiber bundles 60 and 100 to the sensing element tip 75 and reflected by mirror 80. As discussed above, the mirror 80 is mounted on carriage 45 with an angle 3 of about 33.5° to about 37.5° between the mirror 80 and sensing element 110 and in some examples, window 90. As a result, the mirror 80 may direct light 120 through the window with angle of incidence 4 on transparent window 90. Next, the light 123 reflected by the media 150 enters housing 40 through the window 90. Once in the housing 40, light 123 is sensed by the sensing element tip 75 and transmitted through fiber bundles 100 and 60 to the spectrometer 200 on the surface.

In step 740, the spectrometer 200 provides data corresponding the change in the transmitted light 120 and reflected light 123. This data is analyzed by methods known in the art to determine the characteristics of the sensed media 150.

In step 750, the probe 1 may be removed or left in place for on-going NIR sensing.

It will be appreciated by those skilled in the art that changes could be made to the embodiments described above within the scope of the present invention which is defined in the appended claims.

The foregoing has been a detailed description of illustrative embodiments of the disclosure. It is noted that in the present specification conjunctive language such as is used in the phrases "at least one of X, Y and Z" and "one or more of X, Y, and Z," unless specifically stated or indicated otherwise, shall be taken to mean that each item in the conjunctive list can be present in any number exclusive of every other item in the list or in any number in combination with any or all other item(s) in the conjunctive list, each of which may also be present in any number. Applying this general rule, the conjunctive phrases in the foregoing examples in which the conjunctive list consists of X, Y, and Z shall each encompass: one or more of X; one or more of Y; one or more of Z; one or more of X and one or more of Y; one or more of Y and one or more of Z; one or more of X and one or more of Z; and one or more of X, one or more of Y and one or more of Z.

Features of each of the various embodiments described above may be combined with features of other described embodiments as appropriate in order to provide a multiplicity of feature combinations in associated new embodiments. Furthermore, while the foregoing describes a number of separate embodiments, what has been described herein is merely illustrative of the application of the principles of the present disclosure. Additionally, although particular methods herein may be illustrated and/or described as being performed in a specific order, the ordering is highly variable within ordinary skill to achieve aspects of the present disclosure.

Exemplary embodiments have been disclosed above and illustrated in the accompanying drawings. It will be understood by those skilled in the art that various changes, omissions and additions may be made to that which is specifically disclosed herein.

However, the scope of the invention is defined by the appended claims.

## Claims

1. A sensing unit comprising:
a tubular housing (40) including a sidewall (48) extending between a first end (52) and a second end (53);
a transparent window (90) formed in the sidewall (48) of the housing;
a fiber optic bundle (100), comprising least one light emitting fiber (135) configured to emit near infrared light and at least one light collecting fiber (125) configured to collect near infrared light, with a sensing element (110), including a sensing element tip (75), and mounted inside the housing (40);
a mirror (80) disposed in the housing (40) and opposite the sensing element (110), the mirror mounted at a first acute angle (3) with respect to a central longitudinal axis (C3 of the fiber optic bundle (100), the first acute angle designed and configured to cause the near infrared light (120), emitted by the sensing element (110), to be reflected from the mirror (80), to be incident on a surface of the window (90) at a second acute angle (4) with respect to an axis that is normal to the surface of the window (90), and to be transmitted through the transparent window (90);
a conical tip (15) connected to the first end of the housing, the conical tip configured to penetrate a media (150); and
a carriage (45) configured to be slid in the tubular housing, the carriage having an exterior surface (24) abutting the interior housing surface (43);
wherein the carriage (45) includes a mirror recess (83) formed in the exterior surface, the mirror recess defined by a first surface (81) and a second surface (82), the second surface including a mount recess (86) configured to hold the mirror (80) at the first acute angle; and
a sensor mount (38) formed in the carriage (45), the sensor mount configured to hold the sensing element (110) opposite the mirror (80);
wherein the carriage (45) is positioned inside the housing (40) such that the mirror (80) and the sensing element tip (75) are adjacent the transparent window (90).

2. The sensing unit of claim 1, wherein the sensor mount (38) is an elongate longitudinal opening in the carriage (45) that intersects the mirror recess (86) at the first surface (81), the fiber optic bundle (100) being disposed in the elongate longitudinal opening.

3. The sensing unit of claim 2, wherein the tip (75) of the sensing element (110) protrudes from the first surface (81) and is disposed in the mirror recess (86).

4. The sensing unit of claim 1, further comprising a light source (210) external to the housing and coupled to the fiber optic bundle (100).

5. The sensing unit of claim 1, wherein the first acute angle (3) of the mirror (80) with respect to the central longitudinal axis (C3) of the sensing element (110) is in a range from about 33.5 degrees to about 38.5 degrees.

6. The sensing unit of claim 1, wherein the mirror (80) further comprises:
a mounting surface (88) and a reflecting surface,
wherein the transparent window (90) includes first and second parallel surfaces (94, 93),
wherein an angle of the reflecting surface relative to the first and second parallel surfaces of the transparent window is in a range from about 33.5 degrees to about 38.5 degrees.

7. The sensing unit of claim 1, wherein the transparent window (90) is sapphire crystal, the sapphire crystal configured to transmit near infrared light to the media exterior of the housing, and the sapphire crystal configured to transmit light reflected from the media into the housing.

8. The sensing unit of claim 1, wherein the first acute angle (3) of the mirror (80) allows the near infrared light to be transmitted through the sapphire crystal and precludes near infrared light reflected by the sapphire crystal from impinging on the sensing element (110).

9. The sensing unit of claim 1, further comprising:
a central opening (56) within the tubular housing (40), the central opening defined by the sidewall (48), and the central opening extending between the first end (52) and the second end (53), wherein the sensing element (110) and the mirror (80) are mounted within the central opening (56) adjacent the window (90).

10. The sensing unit of claim 1, wherein the at least one light collecting fiber (125) includes a first light collecting fiber for transmitting light having a wavelength below about 1000 nm to a first sensor and a second light collecting fiber for transmitting light above about 1000 nm to a second sensor.

11. The sensing unit of claim 1, wherein the window (90) includes a first surface (94) and a second surface (93), the first surface (94) of the window (90) is facing an interior of the housing (40) and the second surface (93) is substantially aligned with an exterior of the housing, wherein the sensing element (110) is mounted in the housing such that the sensing element is configured to emit near infrared light in a path substantially parallel to the first surface (94).

12. The sensing unit of claim 1, wherein the optical fiber bundle (100) includes a first bundle end (198) and a second bundle end (199); the sensing unit further comprising:
a spectrometer (200) external to the housing (40), the spectrometer connected to the at least one light collecting fiber (125) at the second bundle end (199); and
a light source (210) external to the housing, the light source connected to the at least one light emitting fiber at the second bundle end (199), the light source configured to transmit light to the sensing element (110) through the at least one light emitting fiber (135).

13. The sensing unit of claim 1, wherein the near infrared light is emitted from the sensing element tip (75) and the reflected light is collected, wherein the mirror (80) and sensing element tip (75) are laterally offset from a central longitudinal axis (C1) of the tubular housing (40) and positioned directly laterally adjacent the transparent window (90), and the window defines a space envelope that extends in a lateral direction across a width of the tubular housing (40) and extends in an axial direction from a top edge of the window to a bottom edge of the window, wherein the sensing element tip (75) and the mirror (80) are located within the space envelope.

14. The sensing unit of claim 1, wherein the second acute angle (4) is designed and configured according to a wavelength of the near infrared light and a material type of the window (90) to minimize a reflection of the near infrared light by the window while maximizing a transmission of the near infrared light through the window, wherein a material type is sapphire and the second acute angle (4) is in a range of about 14 degrees to about 18 degrees.

15. The sensing element of any one of claims 1-14, wherein the at least one light emitting fiber (135) is configured to emit one or more of UV light, visible light, and near infrared light and the at least one light collecting fiber (125) is configured to collect one or more of UV light, visible light, and near infrared light for spectral analysis.

## Patentansprüche

1. Eine Sensoreinheit, umfassend:
ein rohrförmiges Gehäuse (40) mit einer Seitenwand (48), die sich zwischen einem ersten Ende (52) und einem zweiten Ende (53) erstreckt;
ein transparentes Fenster (90), das in der Seitenwand (48) des Gehäuses ausgebildet ist; ein Lichtleitfaserbündel (100), das mindestens eine Licht emittierende Faser (135), die so konfiguriert ist, dass sie Nahinfrarotlicht emittiert, und mindestens eine Lichtsammelfaser (125), die so konfiguriert ist, dass sie Nahinfrarotlicht sammelt, umfasst, mit einem Sensorelement (110), das eine Sensorelementspitze (75) aufweist und im Inneren des Gehäuses (40) angebracht ist;
einen Spiegel (80), der im Gehäuse (40) und gegenüber dem Sensorelement (110) angeordnet ist, wobei der Spiegel in einem ersten spitzen Winkel (3) in Bezug auf eine zentrale Längsachse (C3) des Lichtleitfaserbündels (100) angebracht ist, wobei der erste spitze Winkel so ausgelegt und konfiguriert ist, dass das vom Sensorelement (110) emittierte Nahinfrarotlicht (120) vom Spiegel (80) reflektiert wird, auf eine Oberfläche des Fensters (90) in einem zweiten spitzen Winkel (4) in Bezug auf eine Achse auftrifft, die senkrecht zur Oberfläche des Fensters (90) steht, und durch das transparente Fenster (90) hindurchtritt;
eine konische Spitze (15), die mit dem ersten Ende des Gehäuses verbunden ist, wobei die konische Spitze so ausgebildet ist, dass sie in ein Medium (150) eindringt; und
einen Schlitten (45), der so ausgebildet ist, dass er in dem rohrförmigen Gehäuse verschoben werden kann, wobei der Schlitten eine Außenfläche (24) aufweist, die an der Innenfläche (43) des Gehäuses anliegt;
wobei der Schlitten (45) eine in der Außenfläche ausgebildete Spiegelaussparung (83) aufweist, wobei die Spiegelaussparung durch eine erste Fläche (81) und eine zweite Fläche (82) definiert ist, wobei die zweite Fläche eine Befestigungsaussparung (86) aufweist, die so ausgebildet ist, dass sie den Spiegel (80) in dem ersten spitzen Winkel hält; und
eine im Schlitten (45) ausgebildete Sensorhalterung (38), wobei die Sensorhalterung so konfiguriert ist, dass sie das Sensorelement (110) gegenüber dem Spiegel (80) hält;
wobei der Schlitten (45) so im Gehäuse (40) positioniert ist, dass der Spiegel (80) und die Sensorelementspitze (75) an das transparente Fenster (90) angrenzen.

2. Die Sensoreinheit nach Anspruch 1, wobei die Sensorhalterung (38) eine längliche Längsöffnung im Schlitten (45) ist, die die Spiegelaussparung (86) an der ersten Fläche (81) schneidet, wobei das Lichtleitfaserbündel (100) in der länglichen Längsöffnung angeordnet ist.

3. Die Sensoreinheit nach Anspruch 2, wobei die Spitze (75) des Sensorelements (110) aus der ersten Fläche (81) herausragt und in der Spiegelaussparung (86) angeordnet ist.

4. Die Sensoreinheit nach Anspruch 1, ferner umfassend eine Lichtquelle (210) außerhalb des Gehäuses, die mit dem Lichtleitfaserbündel (100) gekoppelt ist.

5. Die Sensoreinheit nach Anspruch 1, wobei der erste spitze Winkel (3) des Spiegels (80) in Bezug auf die zentrale Längsachse (C3) des Sensorelements (110) in einem Bereich von etwa 33,5 Grad bis etwa 38,5 Grad liegt.

6. Die Sensoreinheit nach Anspruch 1, wobei der Spiegel (80) ferner umfasst:
eine Befestigungsfläche (88) und eine reflektierende Fläche,
wobei das transparente Fenster (90) erste und zweite parallele Flächen (94, 93) umfasst, wobei ein Winkel der reflektierenden Fläche relativ zu den ersten und zweiten parallelen Flächen des transparenten Fensters in einem Bereich von etwa 33,5 Grad bis etwa 38,5 Grad liegt.

7. Die Sensoreinheit nach Anspruch 1, wobei das transparente Fenster (90) aus Saphirglas besteht, wobei das Saphirglas so ausgebildet ist, dass es Nahinfrarotlicht Zum Medium außerhalb des Gehäuses durchlässt, und wobei das Saphirglas so ausgebildet ist, dass es von dem Medium reflektiertes Licht in das Gehäuse durchlässt.

8. Die Sensoreinheit nach Anspruch 1, wobei der erste spitze Winkel (3) des Spiegels (80) es ermöglicht, dass das Nahinfrarotlicht durch das Saphirglas hindurchgelassen wird, und verhindert, dass vom Saphirglas reflektiertes Nahinfrarotlicht auf das Sensorelement (110) trifft.

9. Die Sensoreinheit nach Anspruch 1, ferner umfassend:
eine zentrale Öffnung (56) innerhalb des rohrförmigen Gehäuses (40), wobei die zentrale Öffnung durch die Seitenwand (48) begrenzt ist und wobei die zentrale Öffnung sich zwischen dem ersten Ende (52) und dem zweiten Ende (53) erstreckt, wobei das Sensorelement (110) und der Spiegel (80) innerhalb der zentralen Öffnung (56) neben dem Fenster (90) angebracht sind.

10. Die Sensoreinheit nach Anspruch 1, wobei die mindestens eine Lichtsammelfaser (125) eine erste Lichtsammelfaser zum Übertragen von Licht mit einer Wellenlänge unterhalb von etwa 1000 nm an einen ersten Sensor und eine zweite Lichtsammelfaser zum Übertragen von Licht oberhalb von etwa 1000 nm an einen zweiten Sensor umfasst.

11. Die Sensoreinheit nach Anspruch 1, wobei das Fenster (90) eine erste Oberfläche (94) und eine zweite Oberfläche (93) umfasst, wobei die erste Oberfläche (94) des Fensters (90) einem Innenraum des Gehäuses (40) zugewandt ist und die zweite Oberfläche (93) im Wesentlichen an einer Außenseite des Gehäuses ausgerichtet ist, wobei das Sensorelement (110) so in dem Gehäuse angebracht ist, dass das Sensorelement so konfiguriert ist, dass es Nahinfrarotlicht in einem Pfad aussendet, der im Wesentlichen parallel zur ersten Oberfläche (94) verläuft.

12. Die Sensoreinheit nach Anspruch 1, wobei das Lichtleitfaserbündel (100) ein erstes Bündelende (198) und ein zweites Bündelende (199) umfasst; wobei die Sensoreinheit ferner umfasst:
ein Spektrometer (200) außerhalb des Gehäuses (40), wobei das Spektrometer mit der mindestens einen Lichtsammelfaser (125) am zweiten Bündelende (199) verbunden ist; und eine außerhalb des Gehäuses angeordnete Lichtquelle (210), wobei die Lichtquelle mit der mindestens einen Licht emittierenden Faser am zweiten Bündelende (199) verbunden ist, wobei die Lichtquelle so konfiguriert ist, dass sie Licht durch die mindestens eine Licht emittierende Faser (135) an das Sensorelement (110) überträgt.

13. Die Sensoreinheit nach Anspruch 1, wobei das Nahinfrarotlicht von Sensorelementspitze (75) ausgesendet und das reflektierte Licht aufgefangen wird, wobei der Spiegel (80) und die Sensorelementspitze (75) seitlich gegenüber einer zentralen Längsachse (C1) des rohrförmigen Gehäuses (40) versetzt und direkt seitlich neben dem transparenten Fenster (90) angeordnet sind, und das Fenster eine Raumhülle definiert, die sich in seitlicher Richtung über eine Breite des rohrförmigen Gehäuses (40) erstreckt und sich in axialer Richtung von einer Oberkante des Fensters zu einer Unterkante des Fensters erstreckt, wobei sich die Sensorelementspitze (75) und der Spiegel (80) innerhalb der Raumhülle befinden.

14. Die Sensoreinheit nach Anspruch 1, wobei der zweite spitze Winkel (4) entsprechend einer Wellenlänge des Nahinfrarotlichts und einer Materialart des Fensters (90) ausgelegt und konfiguriert ist, um eine Reflexion des Nahinfrarotlichts durch das Fenster zu minimieren und gleichzeitig eine Transmission des Nahinfrarotlichts durch das Fenster zu maximieren, wobei die Materialart Saphir ist und der zweite spitze Winkel (4) in einem Bereich von etwa 14 Grad bis etwa 18 Grad liegt.

15. Sensorelement nach einem der Ansprüche 1 bis 14, wobei die mindestens eine lichtemittierende Faser (135) so ausgelegt ist, dass sie UV-Licht, sichtbares Licht und/oder Nahinfrarotlicht emittiert, und die mindestens eine Lichtsammelfaser (125) so ausgelegt ist, dass sie UV-Licht, sichtbares Licht und/oder Nahinfrarotlicht zur Spektralanalyse sammelt.

## Revendications

1. Unité de détection comprenant:
un boîtier tubulaire (40) comportant une paroi latérale (48) s'étendant entre une première extrémité (52) et une seconde extrémité (53);
une fenêtre transparente (90) formée dans la paroi latérale (48) du boîtier;
un faisceau de fibres optiques (100), comprenant au moins une fibre émettrice de lumière (135) configurée pour émettre de la lumière dans le proche infrarouge et au moins une fibre collectrice de lumière (125) configurée pour collecter de la lumière dans le proche infrarouge, avec un élément de détection (110), comprenant une pointe d'élément de détection (75), et monté à l'intérieur du boîtier (40);
un miroir (80) disposé dans le boîtier (40) et en face de l'élément de détection (110), le miroir étant monté selon un premier angle aigu (3) par rapport à un axe longitudinal central (C3) du faisceau de fibres optiques (100), le premier angle aigu étant conçu et configuré pour faire en sorte que la lumière dans le proche infrarouge (120), émise par l'élément de détection (110) est réfléchie par le miroir (80), est incidente sur une surface de la fenêtre (90) selon un deuxième angle aigu (4) par rapport à un axe perpendiculaire à la surface de la fenêtre (90), et est transmise à travers la fenêtre transparente (90);
une pointe conique (15) reliée à la première extrémité du boîtier, la pointe conique étant configurée pour pénétrer dans un milieu (150); et
un chariot (45) conçu pour coulisser dans le boîtier tubulaire, le chariot présentant une surface extérieure (24) venant en butée contre la surface intérieure (43) du boîtier;
le chariot (45) comportant un évidement de miroir (83) formé dans la surface extérieure, l'évidement de miroir étant défini par une première surface (81) et une deuxième surface (82), la deuxième surface comprenant un évidement de montage (86) configuré pour maintenir le miroir (80) selon le premier angle aigu; et
un support de détecteur (38) formé dans le chariot (45), le support de détecteur étant configuré pour maintenir l'élément de détection (110) en face du miroir (80);
le chariot (45) étant positionné à l'intérieur du boîtier (40) de telle sorte que le miroir (80) et la pointe d'élément de détection (75) sont adjacents à la fenêtre transparente (90).

2. Unité de détection selon la revendication 1, dans laquelle le support de détecteur (38) est une ouverture longitudinale allongée dans le chariot (45) qui croise l'évidement de miroir (86) à la première surface (81), le faisceau de fibres optiques (100) étant disposé dans l'ouverture longitudinale allongée.

3. Unité de détection selon la revendication 2, dans laquelle la pointe (75) de l'élément de détection (110) fait saillie depuis la première surface (81) et est disposée dans l'évidement de miroir (86).

4. Unité de détection selon la revendication 1, comprenant en outre une source de lumière (210) externe au boîtier et couplée au faisceau de fibres optiques (100).

5. Unité de détection selon la revendication 1, dans laquelle le premier angle aigu (3) du miroir (80) par rapport à l'axe longitudinal central (C3) de l'élément de détection (110) est compris dans un intervalle allant d'environ 33,5 degrés à environ 38,5 degrés.

6. Unité de détection selon la revendication 1, dans laquelle le miroir (80) comprend en outre:
une surface de montage (88) et une surface réfléchissante,
dans laquelle la fenêtre transparente (90) comprend première et deuxième surfaces parallèles (94, 93),
dans laquelle un angle de la surface réfléchissante par rapport aux première et deuxième surfaces parallèles de la fenêtre transparente est compris dans un intervalle allant d'environ 33,5 degrés à environ 38,5 degrés.

7. Unité de détection selon la revendication 1, dans laquelle la fenêtre transparente (90) est en cristal de saphir, le cristal de saphir étant configuré pour transmettre la lumière dans le proche infrarouge vers le milieu extérieur au boîtier, et le cristal de saphir étant configuré pour transmettre de la lumière réfléchie par le milieu à l'intérieur du boîtier.

8. Unité de détection selon la revendication 1, dans laquelle le premier angle aigu (3) du miroir (80) permet à la lumière dans le proche infrarouge d'être transmise à travers le cristal de saphir et empêche de la lumière dans le proche infrarouge réfléchie par le cristal de saphir de frapper l'élément de détection (110).

9. Unité de détection selon la revendication 1, comprenant en outre:
une ouverture centrale (56) à l'intérieur du boîtier tubulaire (40), l'ouverture centrale étant définie par la paroi latérale (48), et l'ouverture centrale s'étendant entre la première extrémité (52) et la seconde extrémité (53), dans laquelle l'élément de détection (110) et le miroir (80) sont montés à l'intérieur de l'ouverture centrale (56) à proximité de la fenêtre (90).

10. L'unité de détection selon la revendication 1, dans laquelle la au moins une fibre collectrice de lumière (125) comprend une première fibre collectrice de lumière destinée à transmettre de la lumière ayant une longueur d'onde inférieure à environ 1000 nm vers un premier détecteur et une deuxième fibre collectrice de lumière destinée à transmettre de la lumière au-dessus d'environ 1000 nm vers un deuxième détecteur.

11. Unité de détection selon la revendication 1, dans laquelle la fenêtre (90) comprend une première surface (94) et une deuxième surface (93), la première surface (94) de la fenêtre (90) est tournée vers un intérieur du boîtier (40) et la seconde surface (93) est essentiellement alignée avec un extérieur du boîtier, dans laquelle l'élément de détection (110) est monté dans le boîtier de telle sorte que l'élément de détection est configuré pour émettre de la lumière dans le proche infrarouge selon un trajet essentiellement parallèle à la première surface (94).

12. Unité de détection selon la revendication 1, dans laquelle le faisceau de fibres optiques (100) comprend une première extrémité de faisceau (198) et une seconde extrémité de faisceau (199) ; l'unité de détection comprenant en outre:
un spectromètre (200) externe au boîtier (40), le spectromètre étant connecté à la au moins une fibre collectrice de lumière (125) à la seconde extrémité de faisceau (199); et une source de lumière (210) externe au boîtier, la source de lumière étant connectée à la au moins une fibre émettrice de lumière à la seconde extrémité de faisceau (199), la source de lumière étant configurée pour transmettre de la lumière à l'élément de détection (110) à travers la au moins une fibre émettrice de lumière (135).

13. Unité de détection selon la revendication 1, dans laquelle la lumière dans le proche infrarouge est émise depuis la pointe d'élément de détection (75) et la lumière réfléchie est collectée, dans laquelle le miroir (80) et la pointe d'élément de détection (75) sont décalés latéralement par rapport à un axe longitudinal central (C1) du boîtier tubulaire (40) et positionnés directement adjacents latéralement à la fenêtre transparente (90), et la fenêtre définit une enveloppe spatiale qui s'étend dans une direction latérale sur une largeur du boîtier tubulaire (40) et s'étend dans une direction axiale depuis un bord supérieur de la fenêtre jusqu'à un bord inférieur de la fenêtre, dans laquelle la pointe d'élément de détection (75) et le miroir (80) sont situés à l'intérieur de l'enveloppe spatiale.

14. Unité de détection selon la revendication 1, dans laquelle le deuxième angle aigu (4) est conçu et configuré en fonction d'une longueur d'onde de la lumière dans le proche infrarouge et d'un type de matériau de la fenêtre (90) afin de minimiser une réflexion de la lumière dans le proche infrarouge par la fenêtre tout en maximisant une transmission de la lumière dans le proche infrarouge à travers la fenêtre, le type de matériau étant du saphir et le deuxième angle aigu (4) se situe dans un intervalle d'environ 14 degrés à environ 18 degrés.

15. Élément de détection selon l'une quelconque des revendications 1 à 14, dans lequel la au moins une fibre émettrice de lumière (135) est configurée pour émettre l'une ou plusieurs parmi la lumière UV, la lumière visible et/ou la lumière dans le proche infrarouge, et la au moins une fibre collectrice de lumière (125) est configurée pour collecter l'une ou plusieurs parmi la lumière UV, la lumière visible et/ou la lumière dans le proche infrarouge pour une analyse spectrale.
